# EUROPEAN PATENT SPECIFICATION

(11) **EP 1 617 890 B1**
(45) Date of publication and mention of the grant of the patent: **30.06.2010**
(21) Application number: 04760444.2
(22) Date of filing: 29.04.2004
(51) Int. Cl.: A61M 16/00, A61M 16/04, A61M 5/00, A61F 2/00, A61L 27/00, A61L 29/16, A61L 29/08, A61M 39/00, A61M 5/14

(54) **MEDICAL DEVICE WITH ANTIMICROBIAL LAYER**
MEDIZINISCHE VORRICHTUNG MIT EINER ANTIMIKROBIELLEN SCHICHT
DISPOSITIF MEDICAL A COUCHE ANTIMICROBIENNE

(30) Priority: 29.04.2003 US 425030
(43) Date of publication of application: 25.01.2006
(73) Proprietor: MALLINCKRODT INC., St. Louis, Missouri 63134 (US)
(72) Inventor: MARTENS, Paul, W., Pleasanton, CA 94566 (US); NIETO, Robert, L., Livermore, CA 94550 (US); VIRAG, Robert, Chesterfield, MO 63017 (US); POTTER, Adin M., South Glenn Falls, New York 12803 (US)
(74) Representative: Kinsler, Maureen Catherine
(86) International application number: PCT/US2004/013196
(87) International publication number: WO 2004/096330

(56) References cited:
- EP-A- 0 590 348
- EP-A- 1 270 018
- WO-A-01/43788
- WO-A-02/24246
- US-A- 5 620 738
- US-A1- 2003 031 699
- US-A1- 2003 049 300
- US-A1- 2003 069 541
- US-B1- 6 475 631
- PATENT ABSTRACTS OF JAPAN vol. 2000, no. 16, 8 May 2001 (2001-05-08) & JP 2001 026439 A (TOAGOSEI CO LTD;NGK FRIT CO LTD), 30 January 2001 (2001-01-30)

## Description

### BACKGROUND OF THE INVENTION

### Field of the Invention:

The present invention relates to medical devices, and more particularly, to a method of adding an antimicrobial function to a medical device. A process for applying a lubricious coating to a surface of a substrate is known from US 5620738.

### Description of the Related Art:

Ventilator-associated pneumonia may be a cause of morbidity in critically ill patients. Approximately 250,000 cases of Ventilator Associated Pneumonia (VAP) are a reported each year. The mortality associated with VAP is approximately 23,000 patients annually. (Engelmann, J. et. al.: Ventilator-Associated Pneumonia, Seminars in Infection Control, Vol. 1 No. 2 2001).

Prolongation of hospitalization, ventilation, and management of VAP infections may add up to seven days in additional patient care and over $5,000 in incremental treatment costs per patient. The costs associated with VAP may be in excess of $1.5 billion per year. It would be desirable if costs associated with the prevention and intervention of VAP could be reduced.

VAP may be associated with the long-term use of invasive positive pressure medical devices such as mechanical ventilators or tracheal tubes. Medical devices may be suction catheters, gastric feeding tubes, esophageal obturators, esophageal balloon catheters, oral and nasal airways, bronchoscopes, breathing circuits, filters, heat and moisture exchanges, or humidifiers. Tracheal tubes may be airway management devices such as endotracheal (ET) tubes, tracheostomy tubes, or transtracheal tubes.

Airway management devices such as ET tubes may be associated with VAP because they may provide a substrate upon which bacterial colonization can occur. Bacteria for colonization may come from inhaled aerosols and nasal, oropharyngeal, and gastric secretions. Bacteria for colonization may also result from the formation of microbial adhesions or biofilms on the surfaces of contaminated medical devices. Colonization, in turn, may lead to microaspiration of pulmonary pathogens and related lung infection.

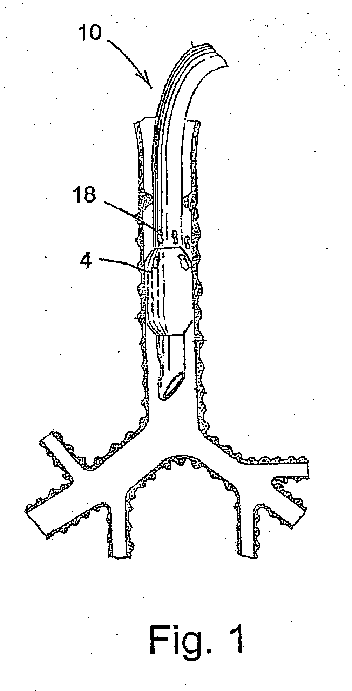

As shown in Fig. 1, bacteria 18 for colonization may "flow" down an ET tube 10 from the mouth along with oral, nasal or gastric secretions. Such bacteria may flow to the area immediately before the cuff 4 and pool there, eventually becoming sessile on the outer surface of the ET tube. Microorganisms may adhere to an abiotic surface and allow complex biofilms to form. The complex biofilm may protect the microorganisms against antibiotic action.

The accretion of antibiotic-resistant biofilms may form a reservoir of infecting microorganisms which may then migrate from the ET outer surface past the protective cuff and contaminate the trachea and lungs. Lung secretions containing microorganisms, blood, mucous, and cellular debris may colonize on the tip and inner lumen of the ET to form biofilms of antibiotic-resistant microorganisms. Such interluminal biofilms may occlude the breathing tube or migrate back into the lungs to cause further infection.

The process of removing these biofilms and secretions with conventional suction catheters may lead to the aspiration of fragments of biofilms or infected aerosols. Contaminated suction catheters, feeding tubes, ventilator tubing and breathing circuits, or filters, heat and moisture exchangers, nebulizers, heated humidifiers, or other related breathing tubes or devices may be sources of microorganism contamination and thus may contribute to biofilm formation.

One method of mitigating colonization of the tube surface by bacteria is by suctioning. Suctioning of subglottic secretions that may collect above the ET cuff may reduce the likelihood of aspiration. Routine suctioning of subglottic secretions may be associated with significant reduction of VAP. The Mallinckrodt Hi-Lo Evac™ tracheal tube is an example of an ET tube with an integral subglottic suctioning apparatus.

Suctioning, aspirating, or draining subglottic secretions, however, requires the frequent intervention of a clinician in order to be effective. It would be desirable if the incidence of VAP could be reduced without extensive reliance on suctioning. It would be desirable if the incidence of VAP could be reduced without requiring additional activities on the part of the clinician in order to be effective.

Another method of mitigating colonization of the tube surface by bacteria is by administration of large doses of antibiotics. Administering large doses of antibiotics, however, may promote the development of more disease resistant bacteriotypes and is thus undesirable.

### SUMMARY

The present invention relates to a medical device according to claim 1, and a method for making the medical device according to claim 17. It is an object of the present invention to reduce the incidence of VAP. This object can be achieved by the features of the independent claims and further enhancements are characterized in the dependent claims. In a first embodiment, a medical device includes a conduit for a fluid which comprises a wall having an outer surface, the wall comprising a hydrophobic polymer, with an outer layer disposed on the outer surface, the outer layer comprising a first quantity of a hydrophilic polymer having an antimicrobial compound substantially dispersed therein, the antimicrobial compound comprising a predetermined amount of phosphorus-based glass having a predetermined quantity of a metal substantially dispersed therein, and wherein the wall and the outer layer are formed by extrusion.

In a second embodiment, a method of making a medical device includes the actions of providing a hydrophobic polymer, extruding the hydrophobic polymer to form a wall, producing an antimicrobial compound comprising a predetermined amount of phosphorus-based glass having a predetermined quantity of a metal substantially dispersed therein, mixing the antimicrobial compound and a hydrophilic polymer, and extruding a layer of the hydrophilic polymer having the antimicrobial compound substantially dispersed therein over an outer surface of the wall.

A system for making a medical device includes means for providing a hydrophobic polymer, means for extruding the hydrophobic polymer to form a wall, means for producing an antimicrobial compound comprising a predetermined amount of phosphorus-based glass having a predetermined quantity of a metal substantially dispersed therein, means for mixing the antimicrobial compound and a hydrophilic polymer, and means for extruding a layer of the hydrophilic polymer having the antimicrobial compound substantially dispersed therein over an outer surface of the wall.

In a fourth embodiment, a medical device includes a conduit for a fluid which comprises a wall having an outer surface, the wall comprising a hydrophobic polymer, with an outer layer disposed on the outer surface, the outer layer comprising a first quantity of a hydrophilic polymer having an antimicrobial compound substantially dispersed therein, the antimicrobial compound comprising a predetermined amount of phosphorus-based glass having a predetermined quantity of a metal substantially dispersed therein, and wherein the wall and the outer layer are formed by molding.

In a fifth embodiment, a method of making a medical device includes the actions of providing a hydrophobic polymer, molding the hydrophobic polymer to form a wall, producing an antimicrobial compound comprising a predetermined amount of phosphorus-based glass having a predetermined quantity of a metal substantially dispersed therein, mixing the antimicrobial compound and a hydrophilic polymer, and molding a layer of the hydrophilic polymer having the antimicrobial compound substantially dispersed therein over an outer surface of the wall.

### BRIEF DESCRIPTION OF THE SEVERAL VIEWS OF THE DRAWINGS

Fig. 1 shows a medical device in situ in a trachea;
Fig. 2 shows a plan view of a medical device according to an embodiment of the invention;
Fig. 3 shows a schematic of an extruder for use with an embodiment of the invention; and
Fig. 4 shows a section of a medical device according to the embodiment of Fig. 2.

### DETAILED DESCRIPTION OF THE PREFERRED EMBODIMENTS

Medical devices are devices which maybe used around or inserted into a living body. One such medical device may be a tube such as an ET tube. Although an ET tube is used in the following examples, the invention is not limited to ET tubes. The invention may apply in various embodiments to other types of medical devices, such as tubes, catheters, stents, feeding tubes, breathing circuits, intravenous tubes, breathing tubes, circuits, and related airway accessories such as connectors, adapters, filters, humidifiers, nebulizers, and prosthetics as well.

Microbes may attach themselves to a surface before beginning colonization of the surface and the formation of biofilms. The colonization of a surface by microbes may require the microbes to become sessile on the surface before attaching themselves to the surface. Preventing the microbes from becoming sessile may thus inhibit the colonization of a surface by microbes.

A biofilm may be described, in general, as a colony cooperative. A biofilm may furthermore be of mixed species with a high degree of specialization or order among individual members of the biofilm. Additionally, normally inactive or quiet genes of these organisms may up-regulate, i.e. turn on, while the organisms settle on a surface, but prior to building the biofilm structure. Up-regulated organisms may become more adaptable to the colony way of life and may also become excessively virulent.

Particles of biofilm may fall into the lungs during treatment. These particles may produce VAP. VAP may also be produced by simple planktonic (free-floating) single cell microbes that may come from leakage around the cuff, or air entering from the tube lumen. Bacteria may become sessile and also resistant to antibiotics by accretion of a protective glycocalyx coating that becomes a biofilm over time. This biofilm may then change its texture, becoming smooth, thus adding further to its defense against antibiotics. Polyvinyl chloride (PVC) may, in some cases, contribute to the formation of such biofilms.

Microbes may be more likely to become sessile on hydrophobic surfaces such as, e.g., those of polyethylene (PE), polypropylene (PP), silicone rubber such as polydimethylsiloxane (PDMS), and PVC. Hydrophobic surfaces reject water. Hydrophilic surfaces, in contrast, which are characterized by an affinity for water, may inhibit microbes from attaching themselves to the surface, and consequently inhibit the formation of biofilms as well.

Friction between a medical device and surrounding tissue may cause irritation in the surrounding tissues such as vocal cords. Such friction may make it more difficult to insert a medical device in the first place- Such friction may also produce trauma to the surrounding tissues. It would be desirable for the surface of a medical device to have lower surface friction, so that it slid across surrounding tissues more easily.

Biofilms may adhere to surfaces of medical devices, resulting in a buildup of dried secretions. It would be desirable for a medical device to have a slippery surface so that biofilms may be less likely to adhere to the surface of the medical device. It would be further desirable for a medical device to have a slippery surface so that biofilms that did build up might be easier to remove by suctioning techniques, so less frequent suctioning might be required.

A medical device such as an ET tube may have a cuff. Such a cuff may form a seal around the tube to block secretions that may otherwise be aspirated. It would be desirable for such a cuff to swell in thickness upon absorbing moisture from the surrounding tissues. It would further be desirable is such a swelling resulted in an ability to seal at a lower pressure, such as a lower contact pressure between the cuff and the surrounding tissues.

Successive concentrations and rarefactions of moisture may occur across a medical device during intubation. It would be desirable if a surface of a medical device could transport moisture across such concentration gradients by, for example, osmosis. It would further be desirable if a hydrophilic layer on an inner diameter of a tracheal tube could condense and absorb moisture from exhaled gases in a cooler region of the tube and re-evaporate the warmed moisture to drier or cooler inhalation gases.

Medical devices, and in particular ET tubes are often formed of hydrophobic materials such as PVC. Microbes may be inhibited from attaching themselves to a hydrophobic surface by applying a hydrophilic layer over the hydrophobic surface. A medical device may be formed of, e.g. a hydrophobic material coated with a hydrophilic material to give it a hydrophilic surface. The hydrophilic coating may be, e.g. a polyurethane (PU), such as medical grade hydrophilic thermoplastic polyurethane. Hydrophilic coatings may be applied by a coating operation.

A medical device, such as an ET tube, may promote respiration. It would be desirable if the carbon dioxide (CO₂) content of respiration could be determined, so as to determine whether the intubation is proper. It would further be desirable if a hydrophilic surface of a medical device were injected with a chemical, such as an acid-base color dye, to indicate CO₂ concentration.

Destroying the microbes before they have a chance to become sessile and colonize the surface of the medical device could mitigate the conditions promoting colonization. It would further be desirable for the incidence of VAP to be reduced without extensive reliance on large doses of antibiotics.

Some elements, such as some metals, may have a deleterious effect on microbes. Some of these metals may be oligodynamic, in that they have an effect in small quantities only. Some metals may kill microbes by destroying their cell walls or by interfering with the metabolic functions of the cells. These metals may thus have antimicrobial or antiseptic properties. Some examples of such metals are copper, silver, or gold. Silver or silver ions (Ag⁺), for example, may be adsorbed on the bacterial cell surface as an RSAg complex-The RSAg complex may immobilize the respiratory activity of the cell and eventually kill the cell.

The hydrophilic layer may therefore further contain a metal such as copper, silver, or gold in a metal bearing material. In several exemplary embodiments, the metal may be elemental silver, powdered silver, silver ions (Ag⁺), or a silver bearing material like silver oxide (AgO). The hydrophilic layer may thus be an antimicrobial (AM) layer. In this way the colonization-inhibiting properties of the hydrophilic surface can be reinforced by antimicrobial properties.

It may be desirable for the silver to be released over time, while the medical device is in use. In one embodiment, therefore, the silver bearing material may be a phosphorus-based glass material that dissolves in water at a rate that may be a function of its particular formulation. The glass may also contain trace amounts of other elements, such as calcium oxide (CaO). The rate at which silver is released may further be a function of the rate at which the phosphorus-based glass material dissolves in water. The silver, or the phosphorus-based glass material, or both may be powdered.

The hydrophilic layer may be wetted with water prior to use. The hydrophilic layer may also attract and absorb water available in the host during use. The absorbed water may then dissolve the silver bearing phosphorus-based glass material and release the silver into the surroundings of the medical device. The rate at which the silver bearing phosphorus-based glass material dissolves in water may in turn be a function of the amount of water available to dissolve it.

The release of silver over time, which is defined as the elution rate and is measured in µ-grams/cm²/day, may thus be tailored to the specific needs of the application by specifying the formulation of the phosphorus-based glass material. In one embodiment, the silver bearing material may be made up of about 5-10 % by weight, e.g. about 7.5 % phosphorus-based glass by weight. Such a material is available from Giltech Limited, 12 North Harbour Industrial Estate, Ayr, Scotland, Great Britain KA8 8BN.

In one embodiment, the elution rate should be up to about 0.01 µ-grams/cm²/day. In another embodiment, the elution rate should be between about 0.01 and 1.0 µ-grams/cm²/day. In a preferred embodiment, the elution rate should be, e.g. about 0.4 µ-grams/cm²/day.

In other embodiments, bioactive pharmaceutical agents such as a bronchodilator, an anti-inflammatory agent, or a local anesthetic may be substantially dispersed in a phosphorus-based glass material within a hydrophilic layer. Such bioactive pharmaceutical agents may be delivered to and absorbed by adjacent tissues in substantially the same manner as silver. Regulation and control of dosage, elution rate, and thickness in substantially the same manner as silver may also provide a beneficial pharmacologic or therapeutic action.

A hydrophilic coating may be applied to the surface of a medical device by, e.g. dipping, spraying, washing, or painting the hydrophilic coating on the surface. Since the volume of a coating is proportional to the thickness of the coating, however, a hydrophilic surface formed in one of these ways may have only a small volume within which silver is retained. Furthermore, if dipping, spraying, washing, or painting formed the hydrophilic coating, the silver may be present only on the surface of the coating.

Since the volume of a coating is necessarily small, the hydrophilic coating may have a limited capacity to hold silver prior to delivery. Furthermore, since the silver may only reside on the surface of the hydrophilic coating, the silver may wash off prematurely, early in the use of the medical device, leaving less silver to prevent future bacteria from becoming sessile and colonizing the surface of the tube.

It would be desirable if a hydrophilic layer were extruded or molded along with the medical device, since controlling the thickness of the extrusion or mold could then optimize the volume of the hydrophilic layer.

In one embodiment, a medical device may be formed by extruding a wall of hydrophobic material along with one or more layers of an AM material. In another embodiment, a medical device may be formed by molding a wall of hydrophobic material along with one or more layers of an AM material. Standard PVC material may form the wall of the medical device, along with one or more layers of an AM material. The AM layer may be formed on an inner or an outer surface of the medical device wall. The AM layer may be comprised of, e.g. polyurethane, such as a medical grade hydrophilic thermoplastic polyurethane into which has been substantially dispersed a silver bearing phosphorus-based glass material.

In one embodiment, the AM layer may be within a range of about 0.002 mm - 2.5 mm in thickness, or about 0.13 mm in thickness. In another embodiment, the AM layer may be within a range of about 0.002 mm - 2.5 mm in thickness. In a third embodiment, the AM layer may be up to about 6.35 mm in thickness. In one embodiment, substantially similar materials may form both the inner and outer surfaces of the tube.

In one embodiment, an inner or an outer AM layer may be simultaneously extruded with the medical device wall in a process commonly known as "co-extrusion". In another embodiment, both an inner and an outer AM layer may be extruded simultaneously with the medical device wall in a process sometimes referred to as "tri-extrusion."

Applying an AM layer to the surface of a medical device may reduce the incidence of VAR There may also be a production cost savings to be gained by extruding an AM layer on a medical device over a conventional coating process.

In one embodiment, an AM layer is also applied to the cuff portion of the medical device. In a preferred embodiment only the outer surface of the cuff will have an AM layer since only the outer surface of the cuff is exposed to the patient. An AM layer may be applied to the outer surface of the cuff portion of the medical device by, e.g. co-extruding the AM layer with the wall of the cuff.

The cuff wall may subsequently be expanded to form a thin-walled cuff device. The cuffwall may be expanded by, e.g. a process such as extrusion blow molding. In this process, a core or mandrel of the extruder has apertures to admit a gas such as pressurized air or an inert gas like nitrogen, into the medical device in the neighborhood of the cuff. After a length of medical device, or parison, has been extruded, a mold clamps the medical device around the mandrel. As gas is admitted to the cuff area through the mandrel the cuff expands against the mold. In the alternative, the cuff wall may be expanded in a second discrete expansion process following an extrusion or molding process, such as with a shuttle blowmolding process.

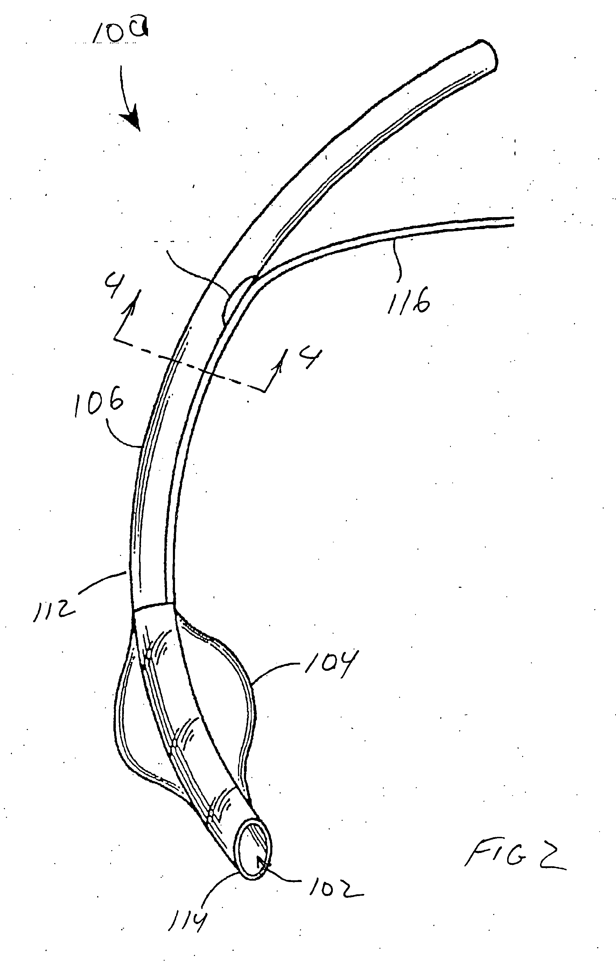

In Fig. 2 is shown a medical device 100 according to a first embodiment of the invention. Medical device 100 may be a catheter, a stent, a feeding tube, an intravenous tube, an ET tube, a circuit, an airway accessory, a connector, an adapter, a filter, a humidifier, a nebulizer, or a prosthetic, in various embodiments.

Medical device 100 may have a conduit 102 for a fluid and an inflatable cuff 104 disposed at a first end 114 of conduit 102. The fluid may be a gas, an aerosol, a suspension, a vapor, or droplets of liquid dispersed in a gas. A lumen 116 may be disposed alongside conduit 102 to inflate cuff 104. In one embodiment, a wall 112 of conduit 102 is made of a hydrophobic polymer, a hydrophilic polymer and an antimicrobial compound.

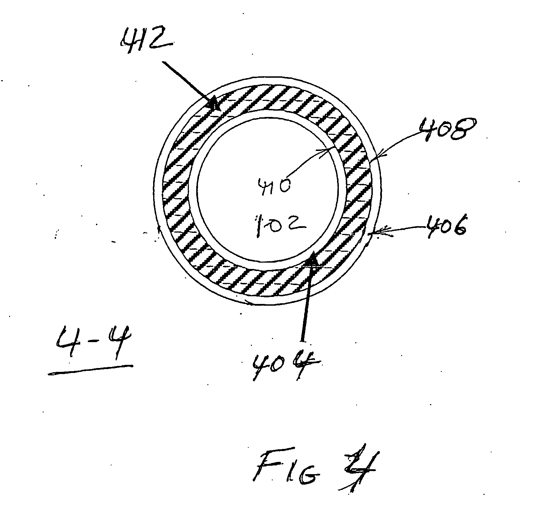

As shown in section 4-4 shown in Fig. 4, a wall 412 of conduit 102 is made of a hydrophobic polymer with an outer layer 406 composed of a hydrophilic polymer and an antimicrobial compound disposed on an outer surface 408 of wall 412. An inner layer 404 composed of a hydrophilic polymer and an antimicrobial compound may further be disposed on an inner surface 410 of wall 412. Outer surface 408 may also be an outer surface of cuff 104.

In one embodiment, wall 412 is a hydrophobic compound containing a hydrophilic polymer and an antimicrobial compound. In one embodiment, a hydrophilic polymer and an antimicrobial compound are substantially dispersed, i.e. mixed with a hydrophobic compound forming wall 412 of conduit 102. In another embodiment, hydrophilic polymer and antimicrobial compound are substantially dispersed within cuff 104, with e.g. a hydrophobic compound forming cuff 104.

In a second embodiment, a method of making a medical device 100 comprises the actions of providing a hydrophobic polymer, a hydrophilic polymer and an antimicrobial compound, combining the hydrophilic polymer and the antimicrobial compound, forming the hydrophobic polymer into a wall 412 of a conduit 102, and substantially simultaneously extruding the hydrophilic polymer and the antimicrobial compound as an outer layer 406 on a outer surface 408 of conduit 102.

In another embodiment, the method further includes forming the hydrophobic polymer into a cuff 104 on an end of conduit 102, and substantially simultaneously extruding the hydrophilic polymer and the antimicrobial compound on a surface of cuff 104.

in another embodiment, the method further includes substantially simultaneously extruding the hydrophilic polymer and the antimicrobial compound as an inner layer 404 on an inner surface 410 of conduit 102 while wall 412 and outer layer 406 are being extruded.

In one embodiment, a resulting thickness of the hydrophilic polymer and the antimicrobial compound layer 404 is controlled by the extruder. In an alternative embodiment, extruding the hydrophobic polymer, the hydrophilic polymer and the antimicrobial compound together forms a wall 412 of conduit 102.

In one embodiment, a wall 412 of conduit 102 may be extruded from a hydrophobic compound while an inner layer 404 is extruded in a first predetermined formulation of a hydrophilic polymer and an antimicrobial compound on an inner surface 410 of conduit 102. In another embodiment, a wall 412 of conduit 102 may be extruded from a hydrophobic compound while an outer layer 406 is extruded in a second predetermined formulation of a hydrophilic polymer and an antimicrobial compound on an outer surface 408 of conduit. In an alternative embodiment, an outer layer 406 composed of a hydrophilic polymer and the antimicrobial compound in a second predetermined Formulation may be, e.g. molded on outer surface 408 of conduit 102. In an alternative embodiment, the hydrophobic polymer, hydrophilic polymer and the antimicrobial compound may be, e.g. compounded together and extruded to form a wall 412 of conduit 102.

In one embodiment, the hydrophobic polymer, hydrophilic polymer and the antimicrobial compound may be, e.g. compounded together and extruded to form a wall 114 of cuff 104. In an alternative embodiment, the hydrophilic polymer and the antimicrobial compound may be, e.g. molded on an outer surface of cuff 104. In an alternative embodiment, the hydrophilic polymer and the antimicrobial compound may be, e.g. extruded on an outer surface of cuff 104. In an alternative embodiment, cuff 104 may be, e.g. formed by extruding the hydrophobic polymer, hydrophilic polymer and antimicrobial compound into a cuff 104, and expanding cuff 104.

A system for making a medical device 100 includes means for providing a hydrophobic polymer, means for extruding the hydrophobic polymer to form a wall 412, means for producing an antimicrobial compound comprising a predetermined amount ofphosphorus-based glass having a quantity of silver substantially dispersed therein, means for mixing the antimicrobial compound and a hydrophilic polymer, and means for extruding an outer layer 406 of the hydrophilic polymer having the antimicrobial compound substantially dispersed therein over an outer surface 408 of the wall 412.

In one embodiment, conduit 102 is formed by molding the hydrophobic polymer, the hydrophilic polymer and the antimicrobial compound in a mold. Either the inner or the outer layers 404, 406, or both, may be molded in a mold with the wall 412. The molding process may be overmolding, insert molding, blow-molding, laminate blow-molding, gas assisted molding, thermoplastic molding, injection molding, or compression molding.

A wall 412 may be formed into a tube covered by either an inner or the outer layers 404, 406 and inserted in a mold. The tube maybe be heated in order to promote conformance to the shape of the mold. A fluid such as pressurized air may be pumped into the tube so that the tube is forced or expanded against an inner surface of the mold. A thickness of layers 404 or 406 may be controlled by a clearance between wall 412 and an inner surface of the mold.

In one embodiment, a wall 412 and either an inner or outer antimicrobial compound layers 404 and 406 may be forced into a mold cavity to form the medical device. In another embodiment, a wall 412 made of hydrophobic polymer is placed in a mold and the hydrophilic polymer and the antimicrobial compound layers 404 and 406 are molded around it.

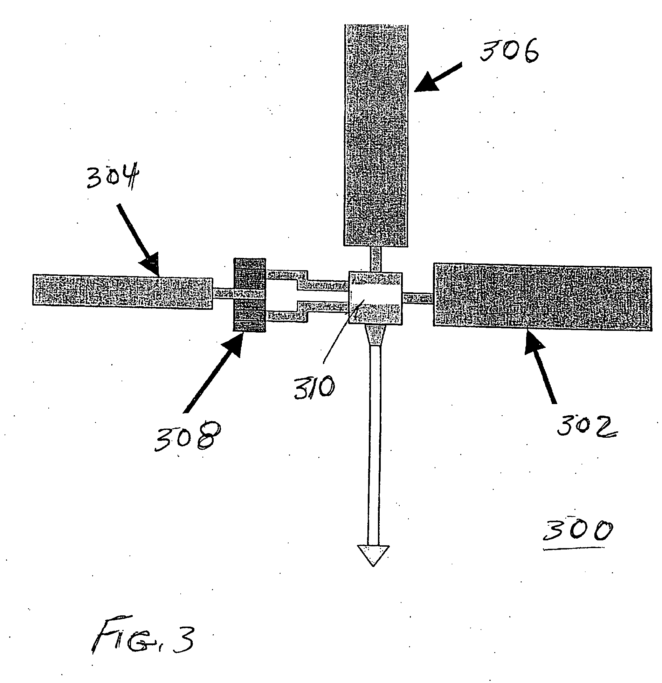

In Fig. 3 is shown an extruder 300 for use with an embodiment of the invention. Fig. 3 may include a main extruder 302 to extrude hydrophobic polymer for the wall 412, a satellite extruder 304 for the AM material, and a satellite extruder 306 for a radio-opaque material. Satellite extruder 304 may feed matching gear pumps 308 to split the AM material into separate layers, one of which may be an inner layer 404 and the other an outer layer 406. A head 310 collects the material streams from the individual satellite extruders, combines them with the flow of material for wall 412 and extrudes them into a medical device 100.

While the invention has been described in detail above, the invention is not intended to be limited to the specific embodiments as described. It is evident that those skilled in the art may now make numerous uses and modifications of and departures from the specific embodiments described herein without departing from the claimed subject-matter.

## Claims

1. A medical device comprising:
a conduit (102) for a fluid;
said conduit (102) comprising a wall (112) having an outer surface (408), said wall (112) comprising a hydrophobic polymer; and
an outer layer (406) disposed on said outer surface (408), said outer layer (406) comprising a first quantity of a hydrophilic polymer having an antimicrobial compound substantially dispersed therein;
**characterized in that** said antimicrobial compound comprises a predetermined amount of phosphorus-based glass having a predetermined quantity of a metal substantially dispersed therein; and
said wall (112) and said outer layer (406) are formed by extrusion or molding.

2. The medical device according to claim 1, wherein said molding is overmolding, insert molding, blow-molding, laminate blow-molding, injection molding, or compression molding.

3. The medical device according to claim 1 or 2, wherein said metal is copper, gold, powdered silver, substantially elemental silver, silver ions, silver oxide, or any combination thereof.

4. The medical device according to claim 1, 2 or 3, wherein said wall (112) further comprises an inner surface (410);
an inner layer (404) disposed on said inner surface (410), said inner layer (404) comprising a second quantity of said hydrophilic polymer having said antimicrobial compound substantially dispersed therein; and
wherein said inner layer (404) is formed by extrusion.

5. The medical device according to claim 4, wherein a thickness of said inner layer (404) is within a range of about 0.002 mm - 2.5 mm.

6. The medical device according to any of the preceding claims, wherein a thickness of said outer layer (406) is within a range of about 0.002 mm - 2.5 mm.

7. The medical device according to any of the preceding claims, wherein a third quantity of said hydrophilic polymer having said antimicrobial compound substantially dispersed therein is substantially dispersed within said wall (112).

8. The medical device according to any of the preceding claims, comprising further a cuff (104) having a cuff outer surface disposed around said medical device, a cuff outer layer disposed on said cuff outer surface, said cuff outer layer comprising a fourth quantity of said hydrophilic polymer having said antimicrobial compound substantially dispersed therein; and
wherein said cuff outer layer is formed by extrusion.

9. The medical device according to any of the preceding claims, wherein said hydrophilic polymer comprises polyurethane or medical grade hydrophilic thermoplastic polyurethane.

10. The medical device according to any of the preceding claims, wherein said metal is releasable from said antimicrobial compound.

11. The medical device according to claim 10, wherein said metal is released at an elution rate of up to about 0.01 µ-grams/cm²/day, at an elution rate of between about 0.01 and about 1.0 µ-grams/cm²/day, or at an elution rate of about 0.4 µ-grams/cm²/day.

12. The medical device according to any of the preceding claims, wherein said predetermined amount of phosphorus-based glass comprises about 0.1 - 50 % of a weight of said antimicrobial compound.

13. The medical device according to any of the preceding claims, wherein said hydrophobic polymer comprises PVC, PE, PU, PDMS, polyester, silicone, or rubber.

14. The medical device according to any of the preceding claims, wherein said medical device comprises an endotracheal tube.

15. The medical device according to any of the preceding claims, wherein said medical device is a catheter, a stent, a feeding tube, a breathing circuit, an intravenous tube, a circuit, an airway accessory, a connector, an adapter, a filter, a humidifier, a nebulizer, or a prosthetic.

16. The medical device according to any of the preceding claims, wherein said antimicrobial compound comprises further an agent in form of an indicator of a carbon dioxide concentration, a bronchodilator, an anti-inflammatory agent, or a local anesthetic.

17. A method of making the medical device of any one of the preceding claims, comprising the steps of:
providing the hydrophobic polymer;
extruding said hydrophobic polymer to form the wall (112) of the conduit (102);
producing the antimicrobial compound comprising a predetermined amount of phosphorus-based glass having a predetermined quantity of a metal substantially dispersed therein;
mixing said antimicrobial compound and a hydrophilic polymer; and
extruding a layer (406) of said hydrophilic polymer having said antimicrobial compound substantially dispersed therein over an outer surface (408) of said wall (112).

18. The method of making the medical device according to claim 17, further comprising the step of extruding a layer (404) of said hydrophilic polymer having said antimicrobial compound substantially dispersed therein over an inner surface (410) of said wall (112).

19. The method of making the medical device according to claim 17 or 18, further comprising the step of forming a cuff (104) on an end of said conduit.

## Patentansprüche

1. Medizinische Vorrichtung, die Folgendes umfasst:
eine Leitung (102) für ein Fluid,
wobei die Leitung (102) eine Wand (112) umfasst, die eine Außenfläche (408) hat, wobei die Wand (112) ein hydrophobes Polymer umfasst, und
eine Außenschicht (406), die auf der Außenfläche (408) angeordnet ist, wobei die Außenschicht (406) eine erste Menge eines hydrophilen Polymers umfasst, das in demselben eine antimikrobielle Zusammensetzung im Wesentlichen dispergiert hat,
**dadurch gekennzeichnet, dass** die antimikrobielle Zusammensetzung eine vorbestimmte Menge eines Glases auf Phosphorbasis umfasst, das in demselben eine vorbestimmte Menge eines Metalls im Wesentlichen dispergiert hat, und
wobei die Wand (112) und die Außenschicht (406) durch Extrudieren oder Formen hergestellt sind.

2. Medizinische Vorrichtung nach Anspruch 1, wobei das Formen Überformen, Einsetzformen, Blasformen, Laminat-Blasformen, Spritzgießen oder Pressformen ist.

3. Medizinische Vorrichtung nach Anspruch 1 oder 2, wobei das Metall Kupfer, Gold, Silberpulver, im Wesentlichen elementares Silber, Silberionen, Silberoxid oder eine beliebige Kombination derselben ist.

4. Medizinische Vorrichtung nach Anspruch 1, 2 oder 3, wobei die Wand (112) ferner Folgendes umfasst: eine Innenfläche (410),
eine Innenschicht (404), die auf der Innenfläche (410) angeordnet ist, wobei die Innenschicht (404) eine zweite Menge des hydrophilen Polymers umfasst, das in demselben die antimikrobielle Zusammensetzung im Wesentlichen dispergiert hat, und
wobei die Innenschicht (404) durch Extrudieren hergestellt ist.

5. Medizinische Vorrichtung nach Anspruch 4, wobei eine Dicke der Innenschicht (404) innerhalb eines Bereichs von etwa 0,002 mm bis 2,5 mm liegt.

6. Medizinische Vorrichtung nach einem der vorhergehenden Ansprüche, wobei eine Dicke der Außenschicht (406) innerhalb eines Bereichs von etwa 0,002 mm bis 2,5 mm liegt.

7. Medizinische Vorrichtung nach einem der vorhergehenden Ansprüche, wobei eine dritte Menge des hydrophilen Polymers, das in demselben die antimikrobielle Zusammensetzung im Wesentlichen dispergiert hat, innerhalb der Wand (112) im Wesentlichen dispergiert ist.

8. Medizinische Vorrichtung nach einem der vorhergehenden Ansprüche, die ferner Folgendes umfasst: eine Manschette (104) mit einer Manschettenaußenfläche, die um die medizinische Vorrichtung angeordnet ist, eine Manschettenaußenschicht, die auf der Manschettenaußenfläche angeordnet ist, wobei die Manschettenaußenschicht eine vierte Menge des hydrophilen Polymers umfasst, das in demselben die antimikrobielle Zusammensetzung im Wesentlichen dispergiert hat, und
wobei die Manschettenaußenschicht durch Extrudieren hergestellt ist.

9. Medizinische Vorrichtung nach einem der vorhergehenden Ansprüche, wobei das hydrophile Polymer Polyurethan oder hydrophiles thermoplastisches Polyurethan medizinischer Güte umfasst.

10. Medizinische Vorrichtung nach einem der vorhergehenden Ansprüche, wobei das Metall aus der antimikrobiellen Zusammensetzung freigesetzt werden kann.

11. Medizinische Vorrichtung nach Anspruch 10, wobei das Metall mit einer Abgabegeschwindigkeit von bis zu etwa 0,01 µ-Gramm/cm²/Tag, mit einer Abgabegeschwindigkeit von zwischen etwa 0,01 und etwa 1,0 µ-Gramm/cm²/Tag oder mit einer Abgabegeschwindigkeit von etwa 0,04 µ-Gramm/cm²/Tag freigesetzt wird.

12. Medizinische Vorrichtung nach einem der vorhergehenden Ansprüche, wobei die vorbestimmte Menge eines Glases auf Phosphorbasis etwa 0,1 bis 50 % eines Gewichts der antimikrobiellen Zusammensetzung umfasst.

13. Medizinische Vorrichtung nach einem der vorhergehenden Ansprüche, wobei das hydrophobe Polymer PVC, PE, PU, PDMS, Polyester, Silikon oder Kautschuk umfasst.

14. Medizinische Vorrichtung nach einem der vorhergehenden Ansprüche, wobei die medizinische Vorrichtung einen Endotrachealtubus umfasst.

15. Medizinische Vorrichtung nach einem der vorhergehenden Ansprüche, wobei die medizinische Vorrichtung ein Katheter, ein Stent, ein Ernährungstubus, ein Beatmungskreissystem, ein intravenöser Tubus, ein Kreissystem, ein Atemweganschlussteil, ein Verbinder, ein Adapter, ein Filter, ein Befeuchter, ein Vernebler oder eine Prothese ist.

16. Medizinische Vorrichtung nach einem der vorhergehenden Ansprüche, wobei die antimikrobielle Zusammensetzung ferner einen Wirkstoff in Form eines Indikators einer Kohlendioxidkonzentration, eines Brochospasmolytikums, eines entzündungshemmenden Wirkstoffs oder eines Lokalanästhetikums umfasst.

17. Verfahren zum Herstellen der medizinischen Vorrichtung nach einem der vorhergehenden Ansprüche, das die folgenden Schritte umfasst:
Bereitstellen des hydrophoben Polymers,
Extrudieren des hydrophoben Polymers, um die Wand (112) der Leitung (102) herzustellen,
Herstellen der antimikrobiellen Zusammensetzung, die eine vorbestimmte Menge eines Glases auf Phosphorbasis umfasst, das in demselben eine vorbestimmte Menge eines Metalls im Wesentlichen dispergiert hat,
Vermischen der antimikrobiellen Zusammensetzung und eines hydrophilen Polymers und
Extrudieren einer Schicht (406) des hydrophilen Polymers, das in demselben die antimikrobielle Zusammensetzung im Wesentlichen dispergiert hat, über eine Außenfläche (408) der Wand (112).

18. Verfahren zum Herstellen der medizinischen Vorrichtung nach Anspruch 17, das ferner den Schritt des Extrudierens einer Schicht (404) des hydrophilen Polymers, das in demselben die antimikrobielle Zusammensetzung im Wesentlichen dispergiert hat, über eine Innenfläche (410) der Wand (112) umfasst.

19. Verfahren zum Herstellen der medizinischen Vorrichtung nach Anspruch 17 oder 18, das ferner den Schritt des Formens einer Manschette (104) an einem Ende der Leitung umfasst.

## Revendications

1. Dispositif médical, comprenant :
un conduit (102) pour un fluide ;
ledit conduit (102) comprenant une paroi (112) comportant une surface externe (408), ladite paroi (112) comprenant un polymère hydrophobe ; et
une couche externe (406) disposée sur ladite surface externe (408), ladite couche externe (406) comprenant une première quantité de polymère hydrophile contenant un composé antimicrobien qui y est pratiquement dispersé ;
**caractérisé en ce que** ledit composé antimicrobien comprend une quantité prédéterminée de verre à base de phosphore, contenant une quantité prédéterminée d'un métal qui y est pratiquement dispersée ; et
ladite paroi (112) et ladite couche externe (406) étant formées par extrusion ou par moulage.

2. Dispositif médical selon la revendication 1, dans lequel ledit moulage est un surmoulage, un moulage par insertion, un moulage par soufflage, un moulage par soufflage de pièces en stratifié, un moulage par injection ou un moulage par compression.

3. Dispositif médical selon les revendications 1 ou 2, dans lequel ledit métal est du cuivre, de l'or, de l'argent sous forme de poudre, de l'argent pratiquement élémentaire, des ions d'argent, de l'oxyde d'argent ou une quelconque combinaison de ces substances.

4. Dispositif médical selon les revendications 1, 2 ou 3, dans lequel ladite paroi (112) comprend en outre une surface interne (410) ;
une couche interne (404) disposée sur ladite surface interne (410), ladite couche interne (404) comprenant une deuxième quantité dudit polymère hydrophile, contenant ledit composé antimicrobien qui y est pratiquement dispersé ; et
ladite couche interne (404) étant formée par extrusion.

5. Dispositif médical selon la revendication 4, dans lequel une épaisseur de ladite couche interne (404) est comprise dans un intervalle allant d'environ 0,002 mm à 2,5 mm.

6. Dispositif médical selon l'une quelconque des revendications précédentes, dans lequel une épaisseur de ladite couche externe (406) est comprise dans un intervalle allant d'environ 0,002 mm à 2,5 mm.

7. Dispositif médical selon l'une quelconque des revendications précédentes, dans lequel une troisième quantité dudit polymère hydrophile contenant ledit composé antimicrobien qui y est pratiquement dispersé est pratiquement dispersée dans ladite paroi (112).

8. Dispositif médical selon l'une quelconque des revendications précédentes, comprenant en outre un manchon (104), comportant une surface externe du manchon disposée autour dudit dispositif médical, une couche externe du manchon disposée sur ladite surface externe du manchon, ladite surface externe du manchon comprenant une quatrième quantité dudit polymère hydrophile contenant ledit composé antimicrobien qui y est pratiquement dispersé ; et
ladite couche externe du manchon étant formée par extrusion.

9. Dispositif médical selon l'une quelconque des revendications précédentes, dans lequel ledit polymère hydrophile comprend du polyuréthane ou un polyuréthane thermoplastique hydrophile de qualité médicale.

10. Dispositif médical selon l'une quelconque des revendications précédentes, dans lequel ledit métal peut être libéré dudit composé antimicrobien.

11. Dispositif médical selon la revendication 10, dans lequel ledit métal est libéré à un taux d'élution atteignant environ 0,01 µ-grammes/cm²/jour, à un taux d'élution compris entre environ 0,01 et environ 1,0 µ-grammes/cm²/jour, ou à un taux d'élution d'environ 0,4 µ-grammes/cm²/jour.

12. Dispositif médical selon l'une quelconque des revendications précédentes, dans lequel ladite quantité prédéterminée de verre à base de phosphore représente environ 0,1 à 50% d'un poids dudit composé antimicrobien.

13. Dispositif médical selon l'une quelconque des revendications précédentes, dans lequel ledit polymère hydrophile comprend du PVC, du PE, du PU, du PDMS, du polyester, de la silicone ou du caoutchouc.

14. Dispositif médical selon l'une quelconque des revendications précédentes, dans lequel ledit dispositif médical comprend un tube endotrachéal

15. Dispositif médical selon l'une quelconque des revendications précédentes, dans lequel ledit dispositif médical est un cathéter, un stent, une sonde d'alimentation, un circuit respiratoire, un tube intraveineux, un circuit, un accessoire des voies aériennes, un raccord, un adaptateur, un filtre, un humidificateur, un atomiseur ou un élément prothétique.

16. Dispositif médical selon l'une quelconque des revendications précédentes, dans lequel ledit composé antimicrobien comprend en outre un agent sous forme d'un indicateur d'une concentration du dioxyde de carbone, d'un broncho-dilatateur, d'un agent anti-inflammatoire ou d'un anesthésique local.

17. Procédé de fabrication du dispositif médical selon l'une quelconque des revendications précédentes, comprenant les étapes ci-dessous :
fourniture du polymère hydrophile ;
extrusion dudit polymère hydrophile pour former la paroi (112) du conduit (102) ;
production du composé antimicrobien comprenant une quantité prédéterminée de verre à base de phosphore contenant une quantité prédéterminée d'un métal qui y est pratiquement dispersée ;
mélange dudit composé antimicrobien et d'un polymère hydrophile ; et
extrusion d'une couche (406) dudit polymère hydrophile contenant ledit composé antimicrobien qui y est pratiquement dispersé au-dessus d'une surface externe (408) de ladite paroi (112).

18. Procédé de fabrication du dispositif médical selon la revendication 17, comprenant en outre l'étape d'extrusion d'une couche (404) dudit polymère hydrophile contenant ledit composé antimicrobien qui y est pratiquement dispersé au-dessus d'une surface interne (410) de ladite paroi (112).

19. Procédé de fabrication du dispositif médical selon les revendications 17 ou 18, comprenant en outre l'étape de formation d'un manchon (104) sur une extrémité dudit conduit.
